# EUROPEAN PATENT SPECIFICATION

(11) **EP 1 951 127 B1**
(45) Date of publication and mention of the grant of the patent: **24.03.2010**
(21) Application number: 06805794.2
(22) Date of filing: 21.09.2006
(51) Int. Cl.: A61B 10/02

(54) **A BIOPSY NEEDLE ASSEMBLY AND A DEVICE FOR TAKING A TISSUE SAMPLE**
BIOPSIE-NADELANORDNUNG UND VORRICHTUNG ZUR ENTNAHME EINER GEWEBEPROBE
ENSEMBLE AIGUILLE DE BIOPSIE ET DISPOSITIF DE PRELEVEMENT DE TISSU

(30) Priority: 07.11.2005 US 733802 P
(43) Date of publication of application: 06.08.2008
(73) Proprietor: Janssens, Jaak Ph., 3500 Hasselt (BE)
(72) Inventor: Janssens, Jaak Ph., 3500 Hasselt (BE)
(74) Representative: Bird, Ariane
(86) International application number: PCT/EP2006/009179
(87) International publication number: WO 2007/051509

(56) References cited:
- US-A- 5 172 701
- US-A- 5 213 110
- US-A- 5 282 476
- US-A1- 2003 114 773

## Description

### Technical field of the invention

The present invention relates to biopsy needles and biopsy needle assemblies and devices for sampling tissue from human or animal body, using such biopsy needles or needle assemblies.

### Background of the invention

Biopsy needle assemblies are already well known in different forms. As an example, US2003/0114773A1 describes a biopsy needle assembly comprising an outer cannula for insertion in a tissue of which the front end has a cutting edge for cutting the tissue sample. The biopsy needle assembly comprises further a hollow biopsy needle located inside and coaxially with the cannula, at the front end being provided with a tissue receiving means, such as a helical screw. The tissue receiving means cooperates with the cutting edge of cannula for receiving cut tissue sample. The biopsy needle assembly further can comprise an inner needle or localisation needle, having a front end and a back end, this inner needle being located inside and coaxially with the hollow biopsy needle. In order to be able to sample tissue, the cannula, hollow biopsy needle and inner needle are mutually axially and rotatably moveable with respect to each other.
It is known to create a vacuum at the end of the biopsy needle when brought into the tissue to be sampled, as e.g. described in US5526822. The biopsy needle assembly is to be connected to an external vacuum- source by means of appropriate tubing. Another device for sampling tissue from human or animal body, using a biopsy needle assembly is shown in US2005/0165328A1.
This biopsy needle, once brought in the tissue to be samples, uses a vacuum pressure-creating device connected to a proximal end of the biopsy needle brought into the tissue. This vacuum attracts the surrounding tissues into the receiving room prior to cutting.
The use of a vacuum unit coupled to the assembly by means of tubing, has the disadvantage that the tubing may hinder the physician in his or her work. The presently known vacuum assisted biopsy needle assemblies also require a significant air volume to be extracted to place the needle cavities under vacuum or underpressure, which air volume is present in the tubing, between the needle and an external vacuum unit, or within a hand held unit.. This requires significant size increase of the vacuum unit. It is also a disadvantage of the presently known vacuum assisted biopsy needle assemblies that an additional manipulation or action of the physician is needed in order to activate the vacuum at the end of the biopsy needle. As medical interventions become more and more complex, such additional action to activate the vacuum may easily be overlooked. It is as well a disadvantage that most often, when a physician is to take several consecutive tissue samples, the biopsy needle assembly is to be brought in as many times as tissue samples are needed. This obviously causes discomfort for both patient and physician, may result in more damage caused to the patient by the consecutive penetration of the body and may cause seeding of diseased, e.g. tumour cells along the extraction pathway.

It is also a disadvantage of presently known assemblies, that driving means such as motors are located in the hand hold device, which may cause interference, such as EMI interference, with the other apparatuses used during the medical manipulation, e.g. scanning equipment such as MRI scanners which are used in conjunction with the biopsy needle to ensure accurate placing of the needle tip.

### Summary of the invention

It is an object of the present invention to provide improved biopsy needle assembly of operating and manufacturing the same. It is an object of the present invention to provide an improved device and method for sampling tissue using a biopsy needle assembly.

In some embodiments of the present invention the biopsy needle assembly can have as an advantage that no tubing, recipients nor electrical vacuum aspirators is used to provide a suction or vacuum pressure to the end of the biopsy needle.

It is also an advantage of some embodiments of the present invention, that the suction or vacuum pressure is created automatically with the normal manipulations of the biopsy needle, i.e. independently from special actions taken by the physician in order to create the suction or vacuum at the end of the biopsy needle.

It is also an advantage of biopsy needle assemblies according to some of the embodiments of the present invention that the volume of trapped air to be removed before suction or vacuum is applied can be reduced. This can result in a vacuum unit of reduced size.

It is an advantage of the present invention that consecutive tissue samples may be taken without the need of removing the biopsy needle assembly after each sample been taken. It is as well an advantage of the present invention that EMI interferences, e.g. from the driving motors may be avoided.

An advantage of the present invention is the combined action of a helix and suction or a vacuum to localize the specimen into the receiving room during and before cutting.

Another advantage is the lack of noise during the biopsy procedure. Normal vacuum assisted biopsies make a suction noise in the tubings and vacuum generator.

The biopsy needle assembly for taking a tissue sample as subject of the present invention is defined in claim 1.

The cannula and the hollow biopsy needle are mutually axially and rotatably moveable. A biopsy needle assembly for taking tissue sample as subject of the present invention is characterised in that the biopsy needle assembly comprises an inner needle having a front end and a back end. This inner needle is located inside and coaxially with the hollow biopsy needle in such a way that the cannula, the hollow biopsy needle and the inner needle are mutually axially moveable with or without rotation. The back end of the inner needle extends from the cavity of the hollow biopsy needle, at the back end of this hollow biopsy needle. The hollow biopsy needle has a cylindrical back end in which the cavity of the hollow biopsy needle extends. The back end of the inner needle is provided with a cylindrical body, which is moveable inside the cylindrical back end of the hollow biopsy needle. The cylindrical back end and the cylindrical body cooperate to provide a piston-cylinder vacuum unit for creating a lowered air pressure in the cavity when the front end of the hollow biopsy needle is axially moved away from the front end of the inner needle. The stroke of the piston in the piston-cylinder vacuum unit is coaxial with the biopsy needle. This makes for a compact design and reduces the distance between the vacuum unit and the end of the needle to a minimum. This reduces the total air volume between the vacuum unit and the needle tip which in turn means that the vacuum unit only has to exhaust a small volume of air. Further the manufacture of the biopsy needle is made simpler and more easily automated, i.e. reduces labour costs. The system doesn't need other accessoires.

As the biopsy needle assembly is inserted up to the tissue to be sampled, the hollow biopsy needle is first axially moved forward into the tissue by a rotation movement. In this way, the front end of the hollow biopsy needle is axially moved away from the front end of the inner needle. As the inner needle is coupled to the piston of the piston-cylinder vacuum unit, and as the axially forward moving hollow biopsy needle is coupled to the cylinder part of this vacuum unit, the volume within the piston-cylinder vacuum unit increases thus sucking air from the needle and creating an underpressure, suction or vacuum at the moment the biopsy needle is introduced in the tissue. Hence, the suction or vacuum is created automatically and no extra intervention of the physician is necessary. Also the amount of suction or vacuum is adapted to the displacement of the hollow biopsy needle. So the risk of applying too much or too little underpressure is minimised. As the inner needle fills part of the cavity of the hollow biopsy needle, the space in which the suction or vacuum is to be created is further minimized. No tubing from an external vacuum unit nor external recipients are required that may disturb the physician in his or her freedom to move and work.

The term "mutually axially moveable" of the cannula, the hollow biopsy needle and the inner needle is to be understood in the sense that the cannula, the hollow biopsy needle and the inner needle may move in axial direction along the common axis, independent from each other when required. Possibly the cannula, the hollow biopsy needle and the inner needle may further rotate round the common axis, independently from each other when required.

Preferably, the inner needle front end is located substantially at the outer end of the hollow biopsy needle when its cylindrical body is at its closest position near the cavity of the hollow biopsy needle. This further reduces the space or volume which is to be brought under vacuum. The inner needle front end, preferably being pointed, is provided out of stainless steel, preferably out of stainless steel of medical grade. The needle point preferentially is made out of stainless steel of medical grade. To locate the point of the needle system, the physician can use clinical palpation, ultrasound, MRI or stereotactic X-rays technology or a combination of these possibilities.

Preferably the cannula comprises at least one aperture in its outer surface for allowing removal the tissue sample from the tissue receiving means of the hollow biopsy needle. The tissue receiving means is brought in front of the aperture by axially withdrawing this hollow biopsy needle within the cannula. In such a way, the tissue once sampled, may be removed from the cannula, without the necessity to remove the cannula out of the tissue or body which is to be sampled, avoiding seeding during repeated insertions. As this removal is not necessary, several consecutive samplings may be done with only one insertion of the biopsy needle assembly. In a more preferred embodiment, two apertures are provided having substantially the dimensions of the tissue receiving means. The two apertures may be located in front of each other at two opposed sides of the cannula surface. The apertures are preferably located near the back end of the cannula. When the tissue receiving means is brought in front of this aperture or apertures, and a marker element can be coupled to the tissue receiving means so allow accurate return of the tissue receiving means to the position of the previous sampling. The markers allow exact location of the place where a tissue was sampled.

The hollow biopsy needle preferably is rotatably mounted around common axis of cannula, hollow biopsy needle and inner needle. Although any kind of hollow biopsy needle may be used, a hollow biopsy needle comprising a spirally shaped tissue receiving means at its tip is a preferred embodiment. Such tissue receiving means, cooperating with a cannula having a cutting edge at its front end, provides tissue samples with reduced damage.

The cylindrical back end of the hollow biopsy needle may be rotated by a suitable drive means such as a friction wheel or gear contacting the cylindrical outer surface and forcing the cylindrical back end to rotate. Preferably however, the cylindrical back end of this rotatable hollow biopsy needle is provided with a non-slip drive means, e.g. a contact wheel or gear for engaging without slippage with a counter wheel or gear in order to rotate the hollow biopsy needle. Most preferred, the contact wheel and counter wheel are gearwheels. All moving parts of the biopsy device may be made of plastic materials to save weight. Alternatively metal parts may be used, e.g. for gear wheels, to provide longer life and to reduce the volume of the parts. Such gear wheels enable provision of a very accurate radial positioning of the hollow biopsy needle. They may also cause the cooperating gear wheel of the cylindrical back end of the hollow biopsy needle to be blocked by blocking the driving mechanism of the driving means, forcing the gear wheels to rotate.

Preferably the inner needle is rotatably mounted around the common axis of cannula, hollow biopsy needle and inner needle. The cylindrical body of the inner needle may be rotated by a drive means such as a wheel or gear contacting the cylindrical body and forcing the cylindrical body to rotate. Preferably however, the cylindrical body of this rotatable inner needle is provided with a non-slip drive means, e.g. a contact wheel or gear for engaging without slippage with a counter wheel or gear in order to rotate the inner needle. Most preferred, the contact wheel and counter wheel are gear wheels.

Such gear wheels enable to provide a very accurate radial positioning of the inner needle. They may also cause the cooperating gear wheel of the cylindrical body of the inner needle to be blocked by blocking the driving mechanism of the driving means, forcing the gear wheels to rotate.

Optionally, the cylindrical body is provided with an outer screw thread, and the inner side of the cylindrical back end of the hollow biopsy needle includes an inner screw thread engaging with the screw thread of the cylindrical body. In order to provide a piston-cylinder vacuum unit, the cylindrical body comprises means to provide an airtight coupling of cylindrical body and cylindrical back end, such as a seal or joint. Such coupling of the cylindrical body of the inner needle with the cylindrical back end of the hollow biopsy needle provides a well defined axial displacement of the inner needle and hollow biopsy needle when one of the two needles is rotated, while the other is in a fixed radial position.

Turning now to the cannula, preferably the cannula is rotatably mounted around common axis of the cannula, hollow biopsy needle and inner needle. Further the cannula is preferably provided with a cylindrical back end. The outer surface of the cylindrical back end of the hollow biopsy needle can preferably be provided with a screw thread, while the inner surface of the cylindrical back end of the cannula includes a screw thread engaging with the screw thread of the outer surface of the cylindrical back end of the hollow biopsy needle. Again, such coupling of the cylindrical back end of the cannula with the cylindrical back end of the hollow biopsy needle provides a well defined axial displacement of the cannula and hollow biopsy needle when the hollow biopsy needle is rotated, while the cannula is in a fixed radial position. In case the cannula is rotatably mounted, such coupling of the cylindrical back end of the cannula with the cylindrical back end of the hollow biopsy needle by means of cooperating screw threads, provides a well defined axial displacement of the cannula and hollow biopsy needle when the cannula is rotated, while the hollow biopsy needle is in a fixed radial position.

The cylindrical back end of the cannula may be rotated by a drive means, e.g. a wheel or gear contacting the cylindrical outer surface and forcing the cylindrical back end to rotate. Preferably however, the cylindrical back end of this rotatable cannula is provided with a non-slip drive means, e.g. a contact wheel or gear for engaging without slippage with a counter wheel or gear in order to rotate the cannula. Most preferred, the contact wheel and counter wheel are gear wheels.

Such gear wheels enable to provide a very accurate radial positioning of the cannula, and may also cause the cooperating gear wheel of the cylindrical back end of the cannula to be blocked by blocking the driving mechanism of the driving means, enforcing the gear wheels to rotate.

Turning now to the second object of the present invention, a device for taking a tissue sample by means of a biopsy needle assembly as described above is provided as defined in claim 14. This device comprises a means for axially moving forwards and backwards the hollow biopsy needle. The device comprises means for axially moving forwards and backwards the cannula, and it comprises a means for preventing the inner needle from moving in an axial direction when the hollow biopsy needle is moved axially. By providing such means, the biopsy needle assembly as described above may, once inserted in the tissue to be sampled, provide several consecutive tissue samples, without the need of withdrawing the biopsy needle assembly each time a sample is taken, and providing the same level of accuracy with regard to the location of the series of sampled tissue.

Preferably, the means for axially moving the hollow biopsy needle forwards and backwards comprises a first driving means coupled to a first and a second wheel, preferably a gear wheel. The first wheel is provided for engaging and rotating the hollow biopsy needle. The second wheel is provided for engaging and rotating simultaneously the hollow biopsy needle and the inner needle. The means for preventing the inner needle from moving in an axial direction when hollow biopsy needle is moved axially, comprises a means for preventing the inner needle from rotating when the first wheel is engaging and rotating the hollow biopsy needle. Especially in the case when the inner needle and the hollow biopsy needle are coupled by means of cooperating and engaging screw treads, this is sufficient to rotate the hollow biopsy needle while the inner needle is blocked. This occurs when the hollow biopsy needle engages and rotates because of being coupled to the first wheel, while the inner needle is prevented from rotating. Due to the cooperating screw threads, the front end of the inner needle and the front end of the hollow biopsy needle move away from each other. Suction or a vacuum at the end of the inner needle is created due to the piston-cylinder vacuum unit according to the invention, while the tissue receiving means provides a tissue sample. When the cannula is moved forward axially, the sampled tissue is cut off by the cutting edge of the cannula.

When the cannula is rotatably mounted around a common axis of the cannula, hollow biopsy needle and inner needle, the means for moving the cannula axially forwards and backwards may comprise a second driving means coupled to a third wheel, preferably a gear wheel, for engaging and rotating the cannula. Especially in the case the cannula and the hollow biopsy needle are coupled by means of cooperating and engaging screw treads, it is sufficient to rotate the cannula while the hollow biopsy needle is blocked. This occurs when the hollow biopsy needle engages the first wheel, which is however blocked because of the driving means which is stays in a fixed position, while the cannula is to rotate. Due to the screw treads cooperating, the front end of the cannula and the front end of the hollow biopsy needle may be brought more close to each other.

Preferably the cannula comprises at least one aperture in its outer surface for removing the tissue sample from the tissue receiving means of the hollow biopsy needle when the tissue receiving means is brought in front of the aperture by axially moving (withdrawing) the hollow biopsy needle inside the cannula. The means for axially moving forwards and backwards the hollow biopsy needle is then adapted to bring the receiving element in front of the aperture.

This may especially realised by a device which further comprises means for axially moving the hollow biopsy needle and the inner needle simultaneously, e.g..a means comprising a drive unit such as a timing or gearing belt and a driving means for moving said timing or gearing belt. By axially displacing the hollow biopsy needle and inner needle simultaneously backwards, for bringing the samples tissue at the height of the aperture, the suction or vacuum at the inner needle front end will remain and the sampled tissue will be fixed in its position until it is presented at the aperture.

Preferably the driving means comprises motors, electric motors especially DC electric motors that may be battery powered.

In an alternative embodiment of the present invention, the driving means are coupled to the wheels or gears by means of a flexible rotative transmission cable connected to remote driving means. Such coupling by flexible transmission cables enables the motors to be located aside away from other devices sensitive to EMI radiation originating from the motors. Any suitable transmission cable can be used that allows three independent movements. For example, the transmission cable can have three layers coaxially mounted on each other. These layers are constructed and mounted with respect to each other so they can rotate and move axially with regard to each other. At the front end of the cable, connectors are provided to lock the needles on their respective cable layers. These connectors are embedded in a cylindrical extension device that can be handheld for easy manipulation. In addition, a flexible electric cable is present to steer the motors in the master device from the handheld cylindrical extension device. This enables the operating physician to be in full control of the movements of the needle set.

Particular and preferred aspects of the invention are set out in the accompanying independent and dependent claims.

Although there has been constant improvement, change and evolution of devices in this field, the present concepts are believed to represent substantial new and novel improvements, including departures from prior practices, resulting in the provision of more efficient, stable and reliable devices of this nature complying with the increased needs of the operating physicians.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is schematically a biopsy needle assembly of an embodiment of the present invention.
Fig. 2 is detailed view of a cannula being part of a biopsy needle assembly of the embodiment of the present invention as shown in Fig. 1
Fig. is detailed view of a hollow biopsy needle being part of a biopsy needle assembly of the embodiment as shown in Fig. 1
Fig. 4 is detailed view of an inner needle being part of a biopsy needle assembly of the embodiment as shown in Fig. 1
Fig. 5 is schematically device for sampling tissue comprising a biopsy needle assembly of the embodiment as shown in Fig. 1
Fig. 6a, Fig. 6b, Fig. 6c and Fig. 6d are schematically views of a series of steps to explain the working of the device for sampling tissue comprising a biopsy needle assembly of the embodiment as in Fig. 5
Fig. 7 is an outer view of the casing of the device of Fig. 5.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled", also used in the claims, should not be interpreted as being restricted to direct connections only. Thus, the scope of the expression "a device A coupled to a device B" should not be limited to devices or systems wherein an output of device A is directly connected to an input of device B. It means that there exists a path between an output of A and an input of B which may be a path including other devices or means. The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

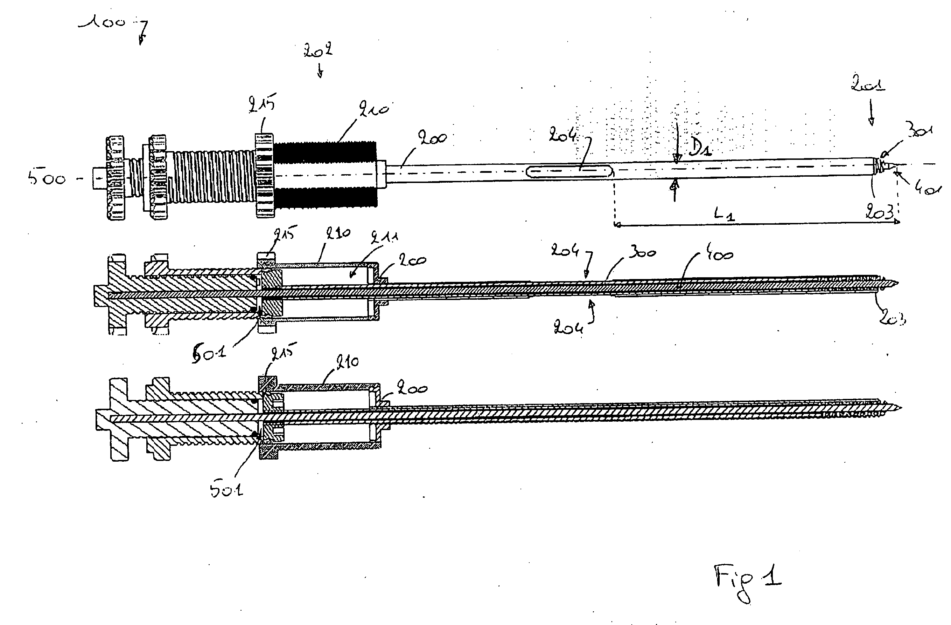

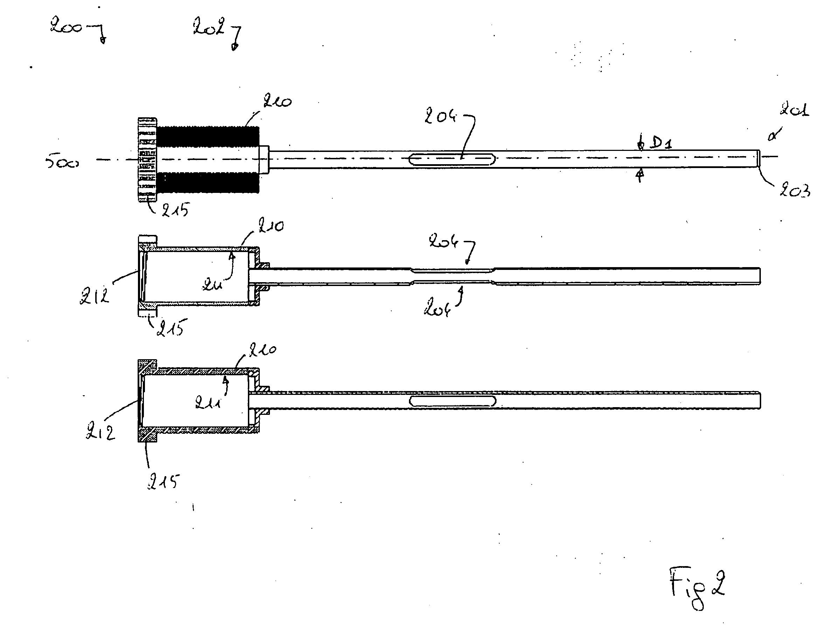

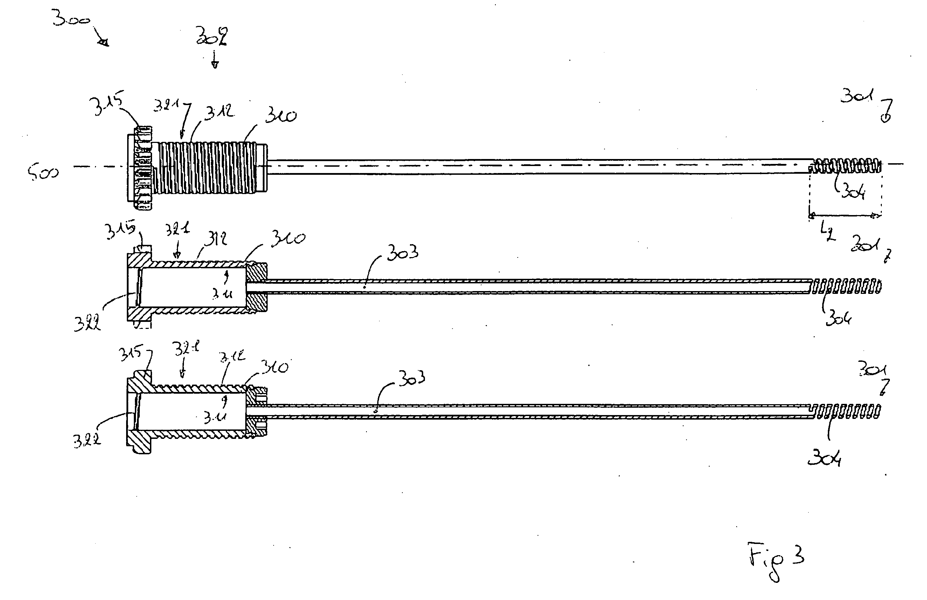

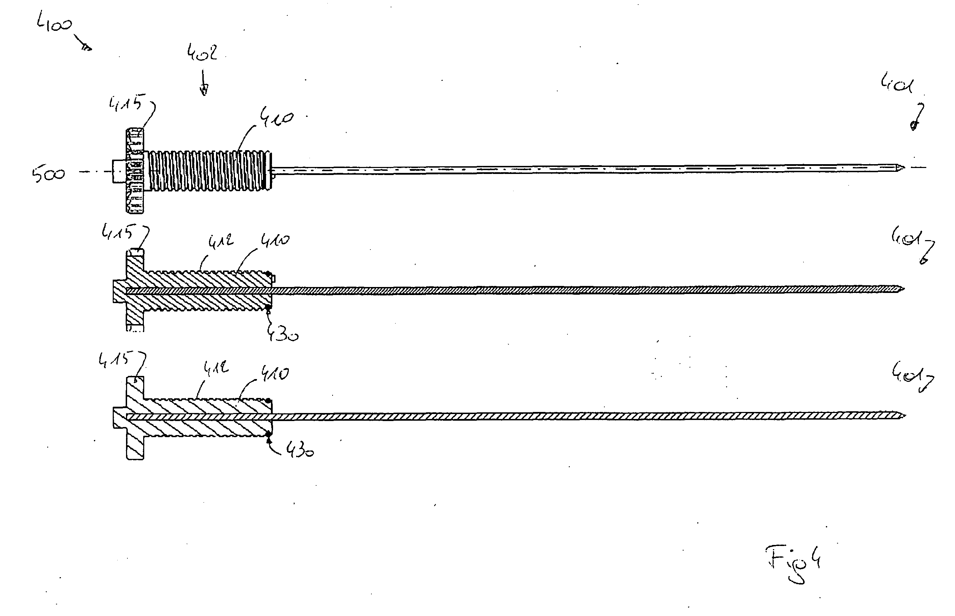

Fig. 1 shows schematically a biopsy needle assembly 100 of an embodiment of the present invention, more in particular a side view and two planar cuts along two mutually perpendicular planes parallel to the axis of the biopsy needle assembly. The biopsy needle assembly comprises three elements: a cannula 200, as shown in similar way in more detail in Fig. 2, a hollow biopsy needle 300 as shown in similar way in more in detail in Fig. 3 and an inner needle 400 as shown in similar way and more in detail in Fig. 4.

The outer cannula 200 has a front end 201 and a back end 202, the front end being for insertion in a tissue such as a patient tissue. The front end has a cutting edge 203 for cutting a tissue sample. The cannula may comprise at least one, or more than one, aperture 204 for removal of the cut sample by the tissue receiving means 304 of the hollow biopsy needle 300. The cannula is provided with a cylindrical back end 210, whose inner side 211 comprises a screw thread 212. The cannula 200 is rotatably mounted around the axis 500 of the biopsy needle assembly. Its cylindrical back end 210 comprises a gear wheel 215 for engaging with a counter gear wheel for making the cannula rotate around the axis 500.
The hollow biopsy needle 300 is located inside and coaxially with the cannula 200. The hollow biopsy needle 300 has an inner cavity 303 and has a front end 301 and a back end 302. The front end 301 is provided with a tissue receiving means 304. Preferably the tissue receiving means 304 is a spirally shaped tissue receiving means, such as disclosed in US 2003/0114773A1. The hollow biopsy needle 300 has a cylindrical back end 310, and the cavity 303 of the hollow biopsy needle 300 extends into this cylindrical back end. Preferably, that hollow biopsy needle is rotatably mounted around the axis 500 of the biopsy needle assembly 100.
The outer surface 321 of the cylindrical back end 310 of the hollow biopsy needle 300 is provided with a screw thread 312 for engaging with the screw thread 212 of the inner side 211 of the cylindrical back end 210 of the cannula 200. The cylindrical back end 310 of the hollow biopsy needle 300 is provided with a gear wheel 315 for engaging with a counter gear wheel in order to rotate the hollow biopsy needle around the axis 500. At the inner side 311 of the cylindrical back end 310 of the hollow biopsy needle 300, a screw thread 322 is provided.
The biopsy needle assembly 100 comprises an inner needle 400 having a front end 401 and a back end 402. The inner needle 400 is located inside and coaxially with the hollow biopsy needle 300. The back end 402 of the inner needle 400 is provided with a cylindrical body 410, which is moveable inside the cylindrical back end 310 of the hollow biopsy needle 300. The inner needle 400 is rotatably mounted around common axis 500 of cannula 200, hollow biopsy needle 300 and inner needle 400

The cylindrical back end and said cylindrical body make up a piston-cylinder vacuum unit for creating a lowered air pressure in said cavity when said front end of said hollow biopsy needle is axially moved away from said front end of said inner needle and the three elements are mutually axially moveable.
The cylindrical body 410 has a screw thread 412, for engaging with the screw thread 322 of said the inner side 311 of said cylindrical back end 310 of said hollow biopsy needle 300. The cylindrical body 410 comprises also means 430 to provide an airtight coupling of the cylindrical body 410 and cylindrical back end 310, e.g. a circular sealing member or joint. The cylindrical body 410 is provided with a gear wheel 415 for engaging with a counter gear wheel in order to rotate said inner needle around the axis 500.
According to the present invention, when the inner needle 400 is rotated relative to the hollow biopsy needle 300, the engagement of the screw thread 322 and screw thread 412 forces the inner needle 400 to move backwards from the hollow biopsy needle 300 or the hollow biopsy needle 300 is forced to move forward from the inner needle 400. Thereby, the inner space 501 is increased in volume and the front end 301 of the hollow biopsy needle 300 is axially moved away from the front end 401 of the inner needle 400. As there is an airtight coupling of the cylindrical body 410 and cylindrical back end 310, a vacuum or lowered air pressure is created in the inner space 501. This lowered air pressure is present in the inner cavity 303 as well, as the cavity 303 extends into this inner space 501. This lowered air pressure, present in the inner cavity 303, creates suction or a vacuum at the tissue receiving means 304, more particular at the location where the tissue receiving means 304 and outer end 401 of the inner needle meet. So, the cylindrical back end 310 and the cylindrical body 410 make up a piston-cylinder vacuum unit for creating a lowered pressure in the cavity 303 when the front end 301 of the hollow biopsy needle 300 is moved away axially from the front end 401 of the inner needle 400.

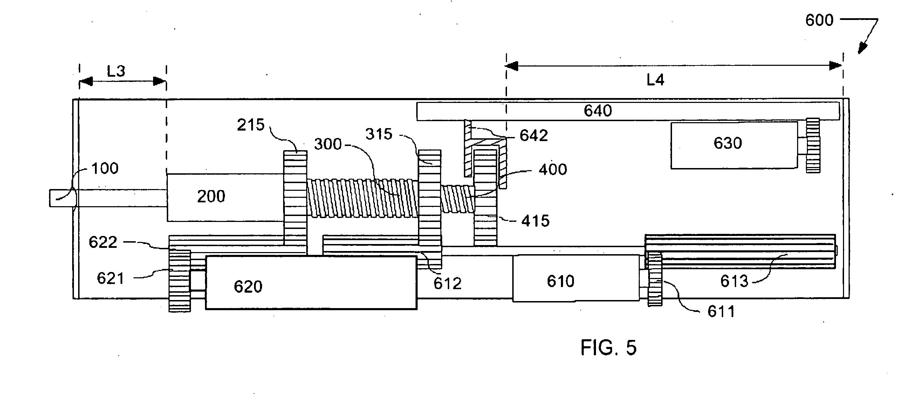

Turning to Fig. 5, a device 600 for taking a tissue sample by means of a biopsy needle assembly 100 is shown. The device 600 comprise means for moving said hollow biopsy needle axially forwards and backwards, being a driving means, which may be a motor 630, which by means of a set of gear wheels and a geared belt 640, may move the hollow biopsy needle as well as the inner needle forward and backward. This means comprises also a driving means, e.g. a motor 610, which drives a gear wheel 611, coupled to only the gear wheel 315 of the cylindrical back end 310 of the hollow biopsy needle 300 by means of a first counter gear wheel 612, or which may engage with the gear Wheel 315 of the cylindrical back end 310 of the hollow biopsy needle 300 and the gear wheel 415 of the inner needle 400 by means of a second counter gear wheel 613. These components, together with appropriate engaging or blocking of gear wheels, and appropriate cooperating of engaging screw treads, as will be explained below, causes the hollow biopsy needle 300 to move forward and backwards in an axial direction as well.

The device 600 comprises means for axially moving the cannula forwards and backwards, these means comprising a driving means, e.g. a motor 620, which drives gear wheel 621 and counter gear wheel 622, the latter engaging with the gear wheel 215 of the cylindrical back end 210 of cannula 200. Together with appropriate engaging or blocking of gear wheels, and appropriate cooperating of engaging screw treads, as will be explained below, causes the cannula to move axially forwards and backwards.

The device comprises means for preventing inner needle to move in axial direction when hollow biopsy needle is axially moved. For this purpose the brake or braking means 642 may be provided. When appropriate cooperating of engaging screw threads, as will be explained below, cause the hollow biopsy needle 300 to move forward and backwards in an axial direction. A brake 642 prevents the inner needle from rotating may also cause the inner needle to be prevented from moving in an axial direction when the hollow biopsy needle is moved axially. The inner needle may be provided as a metal inner needle, e.g. made of stainless steel or a non ferrite metal. It is preferred that the cannula 200, hollow biopsy needle 300 and inner needle 400 as a whole are provided out of stainless steel or other rust-free metal, e.g. medical grade stainless steel.

Preferably, the cylindrical back end 210 and 310 of the cannula and hollow biopsy needle and the cylindrical body 410 of the inner needle are made of a suitable plastic material of which polypropylene is only one example.

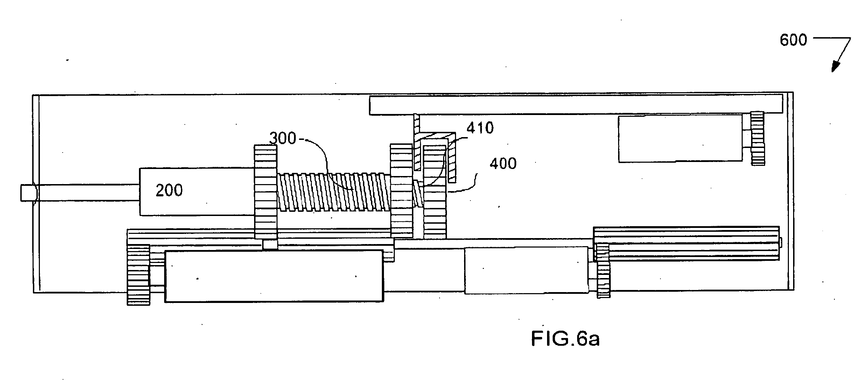

An explanation of how a device 600 with biopsy needle assembly 100 is applied in order to have a tissue sample cut from an organ or other tissue, is now provided and reference is made to Fig. 6a to Fig. 6d, showing the consecutive steps to be applied to sample a tissue.

The device comprises a first motor 610, which drives a gear wheel 611, engaging with only the gear wheel 315 of the cylindrical back end 310 of the hollow biopsy needle 300 by means of a first counter gear wheel 612, or which may engage with the gear wheel 315 of the cylindrical back end 310 of the hollow biopsy needle 300 and the gear wheel 415 of the inner needle 400 by means of a second counter gear wheel 613. The device 600 comprises a second motor 620, which drives gear wheel 621 and counter gear wheel 622, the latter engaging with the gear wheel 215 of the cylindrical back end 210 of cannula 200. The device 600 further comprises a third motor 630, which by means of a set of gear wheels and a geared or timing belt 640, may move the hollow biopsy needle and inner needle forward and backward. The belt is coupled to the gear wheel 415 of the inner needle 400 by means of a brake 642, which may prevent the gear wheel 415 from rotating when the hollow biopsy needle 300 is rotated by engaging with the rotating gear wheel 612.

In Fig. 6a shows the device 600 in a starting position. The inner needle is screwed to its position where the cylindrical body 410 is closest to the cavity 303 of hollow biopsy needle 300. The cylindrical back end 310 of the hollow biopsy needle is positioned in such a way that the screw thread 312 and screw thread 212 are engaged, but in a way that the only the engaging part of screwing thread 312 is located inside the cylindrical body end 210 of the cannula 200. At the front end of the biopsy needle assembly, the cutting edge 203, the front end 301 of hollow biopsy needle and the front end 401 of the inner needle 400 are substantially flush with each other or coterminous. Together they make a point to penetrate the tissues or organs up to the diseased site where a biopsy has to be taken. Preferably, the front end 401 of inner needle 400 is a sharp point, extending slightly out of the cutting edge 203 and front end 301 of hollow biopsy needle 300.

The biopsy needle assembly is inserted in the tissue to be sampled, by penetrating the front end of the biopsy needle assembly into the tissue, until the tissue to be sampled is level with the front end of the biopsy needle assembly. Scanning equipment such as an MRI scanner, CT scanner, stereotactic radiology equipment or ultrasound scanner may be used to assist in locating the needle correctly.

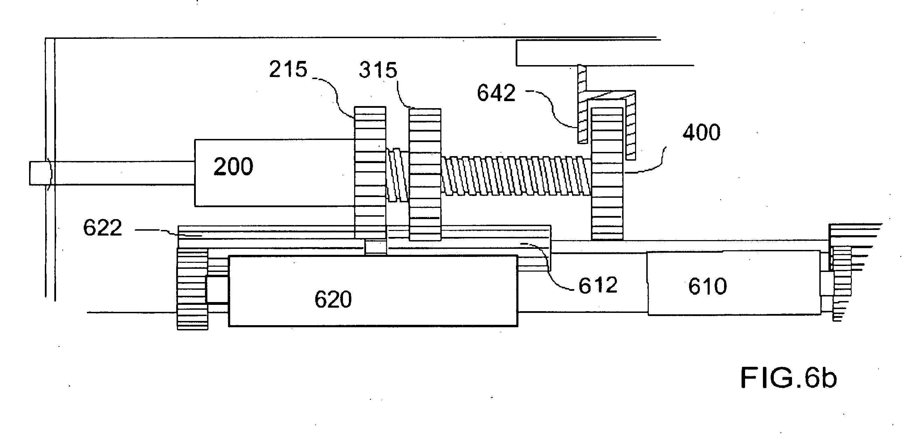

As shown in Fig. 6b, the hollow biopsy needle only is now brought forward. This is done by rotating the hollow biopsy needle 300 by means of engaging the gear wheel 315 with the rotating counter wheel 612, being driven by motor 610. In the mean time, the brake 642 prevents the inner needle from rotating along with the hollow biopsy needle. The engaging screw thread 412 of the inner needle and the screw thread 322 of the cylindrical back end 310 of the hollow biopsy needle 300 force the hollow biopsy needle to move in the direction of the front end. During this movement, the tissue-receiving element 304 is brought, by a screwing action, into the tissue to be sampled. Also during this rotation of the hollow biopsy needle 300, because the inner needle 400 is prevented rotating along with then hollow biopsy needle 300 by a braking force exerted by the braking means 642 on the inner needle 400, the inner needle 400 is prevented from moving axially with the hollow biopsy needle 300. Thus, the inner space 501 is increased in volume and the front end 301 of the hollow biopsy needle 300 is axially moved away from the front end 401 of the inner needle 400, which is penetrating the tissue to be sampled. Via the cavity 303, a vacuum, created in the inner space 501, is provided at the front end of the biopsy needle assembly, where the tissue to be sampled is sucked by the vacuum into the front end 401 of inner needle 400. The cannula 200 is prevented from rotation along with the hollow biopsy needle 300, because the gear wheel 215 is prevented from rotation by engaging with gear counter wheel 622 which is not driven at that moment but remains in a fixed position. Because of the engagement of the screw thread 312 at the outer surface of the cylindrical back end 310 with the screw thread 212 at the inner side 211 of the cylindrical back end 210 of the cannula 200, the cylindrical back end 310 of the hollow biopsy needle 300 is actually screwed into the cylindrical back end 210 of the cannula.

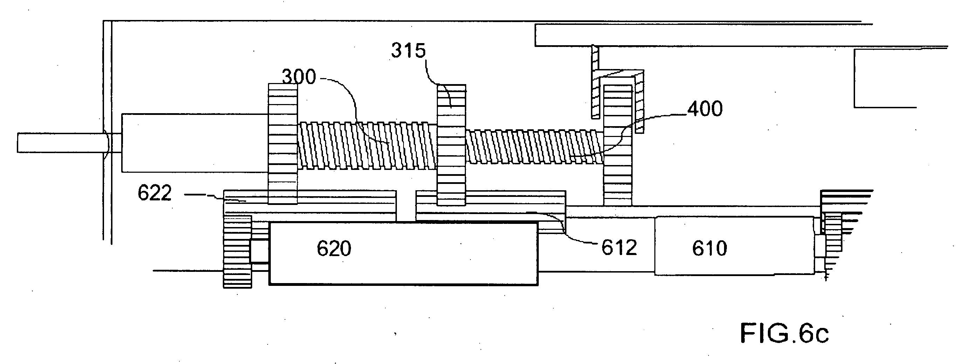

During the next step, as shown in Fig. 6c, the hollow biopsy needle 300 and inner needle 400 are kept in a fixed position by having the gear wheel 315 of the hollow biopsy needle 300 in an engaged position with respect to the counter gear wheel 612, which is not driven. The cannula 200 is rotated by means of the engagement of gear wheel 215 with counter gear wheel 622 driven by motor 620. Because of the engagement of the screwing thread 312 at the outer surface of the cylindrical back end 310 with the screw thread 212 at the inner side 211 of the cylindrical back end 210 of the cannula 200, the cannula is axially moved forwards towards the front end of the tissue receiving means 304, meanwhile cutting the tissue sample which is screwed into the screwing shape of the tissue receiving means. As the hollow biopsy needle 300 and inner needle 400 don't change position one with respect to the other, the vacuum which was created, is maintained and the sampled tissue is kept at the front end 401 of inner needle 400.
The cannula is rotated until the screw thread 312 at the outer surface of the cylindrical back end 310 and the screw thread 212 at the inner side 211 of the cylindrical back end 210 of the cannula 200 disengage. The tissue sample is now present in the tissue receiving means 304.

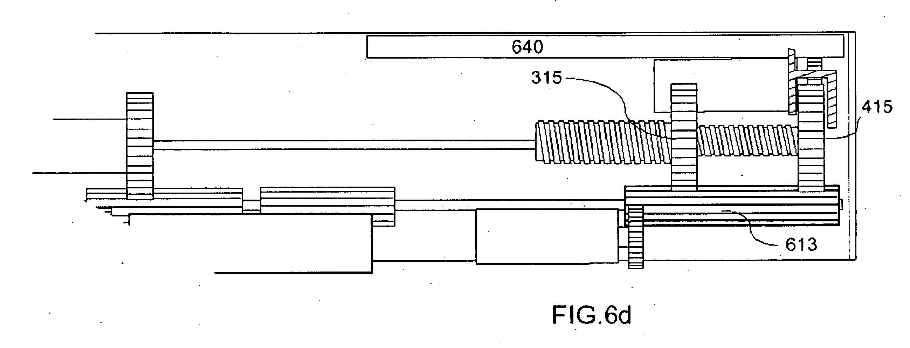

In a next step, as shown in Fig. 6d, the inner needle 400 and hollow biopsy needle 300 are axially moved backwards by the means of a set of gear wheels and a timing or geared belt 640 over such a distance that the sampled tissue in the tissue receiving means is presented at the aperture 204 of the cannula 200, meanwhile bringing gear wheels 315 and 415 in engagement with counter gear wheel 613. By rotation of the gear wheel 613, the inner needle 400 and hollow biopsy needle 300 is rotated simultaneously to line up with the aperture and the tissue can be removed from the receiving means 304. During the removal of the tissue sample, the vacuum is interrupted by air, which is allowed to flow into the cavity and into the inner space 501.

In case another consecutive sample is to be taken, the cannula 200, hollow biopsy needle 300 and inner needle 400 are brought again into their starting position and the above steps may be repeated. The cannula, hollow biopsy needle and inner needle are brought back again by a rotating co-axial movement of the inner needle with the hollow biopsy needle with respect to the cannula. Once the cylindrical element of the hollow biopsy needle meets the cylindrical element of the cannula, the cylindrical elements of both engage. The system searches the reference position to repeat the biopsy procedure.

Possibly a marker element, such as a helix, loop or knot with the external diameter smaller than the internal diameter of the cutting cannula and with an expanding or tissue hooking effect in the target tissue, is provided on the tissue receiving means 304 brought at the height of the aperture or apertures 204. By axially moving forward again of the hollow biopsy needle 300 and inner needle 400, the marker can be used to bring the biopsy needle at the position in the tissue, where the sample of the tissue was taken in previous steps.

As an example, in the biopsy needle assembly 100 as described by means of the figures 1 to 6, following dimensions are used. The cannula 200 has an inner diameter d1 of about 3 to 4 mm. The length of the biopsy needle assembly which can be brought into the tissue to be sampled, this is the length L1 from the front end of the biopsy needle assembly 100 to the aperture 204, is about 100 mm. The length L2 of the tissue receiving means 304, being preferably substantially equal with the length of the aperture or apertures 204 is about 18 to 20 mm. This length L2 is about the length over which the hollow biopsy needle 300 can move axially as compared to fixed cannula 200 and fixed inner needle 400. The length of the above mentioned cooperating screw treads is about L2. The axial displacement L3 that the cannula 200 is allowed to move is about 26 mm

The length of axial displacement L4 of hollow biopsy needle 300 and inner needle 400, in order to provide the sampled tissue before the aperture 204 is about the maximal insertion length L1 of the biopsy needle assembly. In the embodiment as described, L4 is about 100 mm.

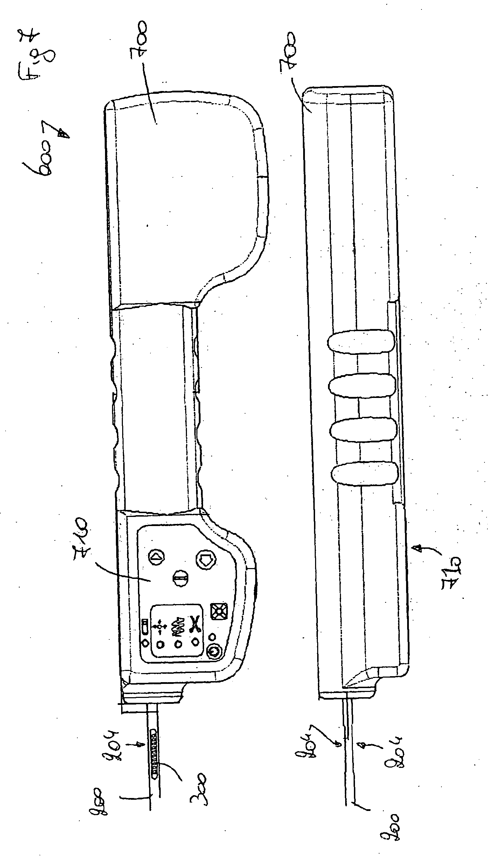

As shown in Fig. 7, in accordance with a further embodiment of the present invention, the device 600 is provided with a hand held casing 700 which may comprise all above mentioned elements, and which has a control panel 710 for controlling the manipulations of the device. The control panel has activating elements such as buttons for initiating and stopping the various manipulations described above, e.g. to start and stop the various motors.

In a further embodiment the different motors 610, 620, and 630 are placed in a separate device, being installed at a position where it cannot cause interference with devices carried by the operating physician or paramedics, other medical or other equipments. The motors are coupled to the gear wheels 611, 612 and gearing to timing belt 640 by means of a flexible rotative transmission cable such as three fold independently layered tubings.

It is to be understood that although preferred embodiments, specific constructions and configurations, as well as materials, have been discussed herein for devices according to the present invention, various changes or modifications in form and detail may be made without departing from the scope of this invention.

## Claims

1. A biopsy needle assembly (100) for taking tissue sample comprising
• an outer cannula (200) having a front end (201) and a back end (202), said front end (201) being for insertion in a tissue, said front end (201) having a cutting edge (203) for cutting a tissue sample;
• a hollow biopsy needle (300) located inside and coaxially with said cannula (200), said hollow biopsy needle (300) having an inner cavity (303) and having a front end (301) and a back end (302), said front end (301) being provided with a tissue receiving means (304), said tissue receiving means (304) configured to co-operate with said cutting edge (203) of cannula (200) for receiving a cut tissue sample, said back end (302) being cylindrical;
• an inner needle (400) having a front end (401) and a back end (402), said inner needle (400) being located inside and coaxially with said hollow biopsy needle (300), said back end (402) of said inner needle (400) being provided with a cylindrical body (410);
• said cannula (200) wand hollow biopsy needle (300) being mutually axially moveable,
**characterised in that** said biopsy needle assembly (100) further comprises a vacuum unit coupled to said hollow biopsy needle (300),
wherein said vacuum unit is a piston cylinder vacuum unit made up from said cylindrical back end (302, 310) and said cylindrical body (410),
wherein said cylindrical body (410) is moveable inside said cylindrical back end (302, 310) of said hollow biopsy needle (300);
wherein the vacuum unit is adapted to create a lowered air pressure in said inner cavity (303) when said front end (301) of said hollow biopsy needle (300) is axially moved away from said front end (401) and in a forward direction relative to said front end (401) of said inner needle, the vacuum unit being located uniaxially with the cannula (200) and hollow biopsy needle (300).

2. A biopsy needle assembly (100) according to claim 1, wherein said cannula (200) and hollow biopsy needle (300) and inner needle (400) are mutually axially moveable, said back end (402) of said inner needle (400) extending from said cavity (303) of said hollow biopsy needle (300) at the back end (302) of said hollow biopsy needle (300), said cavity (303) of said hollow biopsy needle (300) extending in said cylindrical back end (310).

3. A biopsy needle assembly (100) according to claim 2, wherein said inner needle front end (401) is substantially located at the outer end of said hollow biopsy needle (300) when said cylindrical body (410) is at its closest position near said cavity (303).

4. A biopsy needle assembly (100) as in any one of the claims 1 to 3, wherein said cannula (200) comprises at least one aperture (204) in its outer surface for removing said tissue sample from said tissue receiving means (304) of said hollow biopsy needle (300) when said tissue receiving means (304) is brought in front of the aperture (204) by axially moving said hollow biopsy needle (300) inside said cannula (200).

5. A biopsy needle assembly (100) according to any one of the claims 1 to 4, wherein said hollow biopsy needle (300) is rotatably mounted around common axis (500) of cannula (200), hollow biopsy needle (300) and inner needle (400).

6. A biopsy needle assembly (100) according to claim 5, wherein said cylindrical back end (310) of said hollow biopsy needle (300) is provided with a contact wheel (315) for engaging with a counter wheel in order to rotate said hollow biopsy needle (300).

7. A biopsy needle assembly (100) according to any claim 5 or 6, wherein said tissue receiving means (304) is a spirally shaped tissue receiving means (304).

8. A biopsy needle assembly (100) according to any one of the claims 5 to 7, wherein said inner needle (400) is rotatably mounted around common axis (500) of cannula (200), hollow biopsy needle (300) and inner needle (400).

9. A biopsy needle assembly (100) as in claim 8, wherein said cylindrical body (410) having a screw thread (412), the inner side (311) of said cylindrical back end (310)of said hollow biopsy needle (300) comprising a screw thread (322) engaging with said screw thread (412) of said cylindrical body (410), said cylindrical body (410) comprising means (430) to provide an airtight coupling of cylindrical body (410) and cylindrical back end (310).

10. A biopsy needle assembly (100) according to claim 8 or 9, wherein said cylindrical body (410) is provided with a contact wheel (415) for engaging with a counter wheel in order to rotate said inner needle (400).

11. A biopsy needle assembly (100) according to any one of the claims 1 to 10, wherein said cannula (200) is provided with a cylindrical back end (210), the outer surface of said cylindrical back end (310) of said hollow biopsy needle (300) being provided with a screw thread (312), the inner side of said cylindrical back end (210) of said cannula (200) comprising a screw thread (212) engaging with said screw thread (312) of said outer surface of said cylindrical back end (310) of said hollow biopsy needle (300).

12. A biopsy needle assembly (100) according to claim 11, wherein said cannula (200) is rotatably mounted around common axis (500) of cannula (200), hollow biopsy needle (300) and inner needle (400).

13. A biopsy needle assembly (100) according to claim 12, wherein said cylindrical body of said outer surface of said cylindrical back end (210) of said cannula (200) is provided with a contact wheel (215) for engaging with a counter wheel in order to rotate said cannula (200).

14. A device (600) for taking a tissue sample by means of a biopsy needle assembly (100) as in any one of the claims 1 to 13, wherein said device (600) comprises means for axially moving forwards and backwards said hollow biopsy needle (300), said device (600) comprising means for axially moving forwards and backwards said cannula (200), said device comprising means for preventing inner needle (400) to move in axial direction when hollow biopsy needle (300) is axially moved.

15. A device (600) as in claim 14 for taking a tissue sample by means of a biopsy needle assembly (100) in which said means for axially moving forwards and backwards said hollow biopsy needle comprises a first driving means coupled to a first and a second counter wheel, said first counter wheel being provided for engaging and rotating said hollow biopsy needle (300), said second counter Wheel being provided for engaging and rotating simultaneously said hollow biopsy needle (300) and said inner needle (400), said means for preventing said inner needle (400) to move in axial direction when hollow biopsy needle (300) is axially moved comprises a means for preventing said inner needle (400) to rotate when said first counter wheel engaging and rotating said hollow biopsy needle (300).

## Patentansprüche

1. Biopsienadelanordnung (100) zur Entnahme einer Gewebeprobe, umfassend
- eine äußere Kanüle (200) mit einem vorderen Ende (201) und einem hintern Ende (202), wobei das vordere Ende (201) zum Einsetzen in ein Gewebe dient, wobei das vordere Ende (201) eine Schneidkante (203) zum Schneiden einer Gewebeprobe aufweist;
- eine hohle Biopsienadel (300), die im Inneren und koaxial mit der Kanüle (200) angeordnet ist, wobei die hohle Biopsienadel (300) einen inneren Hohlraum (303) aufweist und ein vorderes Ende (301) und ein hinteres Ende (302) aufweist, wobei das vordere Ende (301) mit einem Gewebeaufnahmemittel (304) bereitgestellt ist,
wobei das Gewebeaufnahmemittel (304) so konfiguriert ist, dass es mit der Schneidkante (203) der Kanüle (200) zur Aufnahme einer geschnittenen Gewebeprobe zusammenwirkt, wobei das hintere Ende (302) zylindrisch ist;
- eine innere Nadel (400) mit einem vorderen Ende (401) und einem hinteren Ende (402), wobei die innere Nadel (400) im Inneren und koaxial mit der hohlen Biopsienadel (300) angeordnet ist, wobei das hintere Ende (402) der inneren Nadel (400) mit einem zylindrischen Körper (410) bereitgestellt ist;
- wobei die Kanüle (200) der hohlen Biopsienadel (300) wechselseitig axial beweglich ist,
**dadurch gekennzeichnet, dass** die Biopsienadelanordnung (100) des Weiteren eine Vakuumeinheit umfasst, die an die hohle Biopsienadel (300) gekoppelt ist, wobei die Vakuumeinheit eine Kolbenzylinder-Vakuumeinheit ist, die aus dem zylindrischen hinteren Ende (302, 310) und dem zylindrischen Körper (410) besteht, wobei der zylindrische Körper (410) im Inneren des zylindrischen hinteren Endes (302, 310) der hohlen Biopsienadel (300) bewegbar ist;
wobei die Vakuumeinheit dazu ausgebildet ist, einen verringerten Luftdruck in dem inneren Hohlraum (303) zu erzeugen, wenn das vordere Ende (301) der hohlen Biopsienadel (300) axial von dem vorderen Ende (401) weg und in eine Vorwärtsrichtung relativ zu dem vorderen Ende (401) der inneren Nadel bewegt wird, wobei die Vakuumeinheit uniaxial mit der Kanüle (200) und der hohlen Biopsienadel (300) angeordnet ist.

2. Biopsienadelanordnung (100) nach Anspruch 1, wobei die Kanüle (200) und die hohle Biopsienadel (300) und die innere Nadel (400) wechselseitig axial beweglich sind, wobei das hintere Ende (402) der inneren Nadel (400) von dem Hohlraum (303) der hohlen Biopsienadel (300) am hinteren Ende (302) der hohlen Biopsienadel (300) absteht, wobei sich der Hohlraum (303) der hohlen Biopsienadel (300) in das zylindrische hintere Ende (310) erstreckt.

3. Biopsienadelanordnung (100) nach Anspruch 2, wobei das vordere Ende (401) der inneren Nadel im Wesentlichen an dem äußere Ende der hohlen Biopsienadel (300) angeordnet ist, wenn sich der zylindrische Körper (410) an seiner nächsten Position nahe dem Hohlraum (303) befindet.

4. Biopsienadelanordnung (100) nach einem der Ansprüche 1 bis 3, wobei die Kanüle (200) mindestens eine Öffnung (204) in ihrer Außenfläche zum Entfernen der Gewebeprobe von der Gewebeaufnahmenadel (304) der hohlen Biopsienadel (300) umfasst, wenn das Gewebeaufnahmemittel (304) durch axiales Bewegen der hohlen Biopsienadel (300) im Inneren der Kanüle (200) vor die Öffnung (204) gebracht wird.

5. Biopsienadelanordnung (100) nach einem der Ansprüche 1 bis 4, wobei die hohle Biopsienadel (300) drehbar um eine gemeinsame Achse (500) der Kanüle (200), der hohlen Biopsienadel (300) und der inneren Nadel (400) montiert ist.

6. Biopsienadelanordnung (100) nach Anspruch 5, wobei das zylindrische hintere Ende (310) der hohlen Biopsienadel (300) mit einem Kontaktrad (315) für den Eingriff in ein Gegenrad bereitgestellt ist, um die hohle Biopsienadel (300) zu drehen.

7. Biopsienadelanordnung (100) nach Anspruch 5 oder 6, wobei das Gewebeaufnahmemittel (304) ein spiralförmiges Gewebeaufnahmemittel (304) ist.

8. Biopsienadelanordnung (100) nach einem der Ansprüche 5 bis 7, wobei die innere Nadel (400) drehbar um eine gemeinsame Achse (500) der Kanüle (200), der hohlen Biopsienadel (300) und der inneren Nadel (400) montiert ist.

9. Biopsienadelanordnung (100) nach Anspruch 8, wobei der zylindrische Körper (410) ein Schraubengewinde (412) aufweist, die innere Seite (311) des zylindrischen hinteren Endes (310) der hohlen Biopsienadel (300) ein Schraubengewinde (322) umfasst, das mit dem Schraubengewinde (412) des zylindrischen Körpers (410) in Eingriff gelangt, wobei der zylindrische Körper (410) Mittel (430) zum Bereitstellen einer luftdichten Kopplung des zylindrischen Körpers (410) und des zylindrischen hinteren Endes (310) umfasst.

10. Biopsienadelanordnung (100) nach Anspruch 8 oder 9, wobei der zylindrische Körper (410) mit einem Kontaktrad (415) für den Eingriff in ein Gegenrad bereitgestellt ist, um die innere Nadel (400) zu drehen.

11. Biopsienadelanordnung (100) nach einem der Ansprüche 1 bis 10, wobei die Kanüle (200) mit einem zylindrischen hinteren Ende (210) bereitgestellt ist, wobei die Außenfläche des zylindrischen hinteren Endes (310) der hohlen Biopsienadel (300) mit einem Schraubengewinde (312) bereitgestellt ist, wobei die Innenseite des zylindrischen hinteren Endes (210) der Kanüle (200) ein Schraubengewinde (212) umfasst, das mit dem Schraubengewinde (312) der Außenfläche des zylindrischen hinteren Endes (310) der hohlen Biopsienadel (300) in Eingriff gelangt.

12. Biopsienadelanordnung (100) nach Anspruch 11, wobei die Kanüle (200) drehbar um eine gemeinsame Achse (500) der Kanüle (200), der hohlen Biopsienadel (300) und der inneren Nadel (400) montiert ist.

13. Biopsienadelanordnung (100) nach Anspruch 12, wobei der zylindrische Körper der Außenfläche des zylindrischen hinteren Endes (210) der Kanüle (200) mit einem Kontaktrad (215) für den Eingriff mit einem Gegenrad bereitgestellt ist, um die Kanüle (200) zu drehen.

14. Vorrichtung (600) zum Entnehmen einer Gewebeprobe mit Hilfe einer Biopsienadelanordnung (100) nach einem der Ansprüche 1 bis 13, wobei die Vorrichtung (600) Mittel zum axialen Vorwärts- und Rückwärtsbewegen der hohlen Biopsienadel (300) umfasst, wobei die Vorrichtung (600) Mittel zum axialen Vorwärts- und Rückwärtsbewegen der Kanüle (200) umfasst, wobei die Vorrichtung Mittel zum Verhindern, dass sich die innere Nadel (400) in die axiale Richtung bewegt, wenn die hohle Biopsienadel (300) axial bewegt wird, umfasst.

15. Vorrichtung (600) nach Anspruch 14, zum Entnehmen einer Gewebeprobe mit Hilfe einer Biopsienadelanordnung (100) in der das Mittel zum axialen Vorwärts- und Rückwärtsbewegen der hohlen Biopsienadel ein erstes Antriebsmittel umfasst, das an ein erstes und ein zweites Gegenrad gekoppelt ist, wobei das erste Gegenrad für einen Eingriff mit und zum Drehen der hohlen Biopsienadel (300) bereitgestellt ist, wobei das zweite Gegenrad für den Eingriff mit und zum gleichzeitigen Drehen der hohlen Biopsienadel (300) und der inneren Nadel (400) bereitgestellt ist, wobei das Mittel zum Verhindern, dass sich die innere Nadel (400) in die axiale Richtung dreht, wenn die hohle Biopsienadel (300) axial bewegt wird, ein Mittel zum Verhindern umfasst, dass sich die innere Nadel (400) dreht, wenn das erste Gegenrad mit der hohlen Biopsienadel (300) in Eingriff steht und diese dreht.

## Revendications

1. Ensemble formant aiguille de biopsie (100) pour prendre un échantillon de tissu, comprenant :
• une canule extérieure (200) ayant une extrémité avant (201) et une extrémité arrière (202), ladite extrémité avant (201) servant pour insertion dans un tissu, ladite extrémité avant (201) ayant un bord de coupe (203) pour couper un échantillon de tissu ;
• une aiguille de biopsie creuse (300) située à l'intérieur de ladite canule (200) et coaxiale avec cette dernière, ladite aiguille de biopsie creuse (300) ayant une cavité intérieure (303) et ayant une extrémité avant (301) et une extrémité arrière (302), ladite extrémité avant (301) étant munie d'un moyen de réception de tissu (304), ledit moyen de réception de tissu (304) étant configuré pour coopérer avec ledit bord de coupe (203) de canule (200) pour recevoir un échantillon de tissu coupé, ladite extrémité arrière (302) étant cylindrique ;
• une aiguille intérieure (400) ayant une extrémité avant (401) et une extrémité arrière (402), ladite aiguille intérieure (400) étant située à l'intérieur de ladite aiguille de biopsie creuse (300) et coaxiale avec cette dernière, ladite extrémité arrière (402) de ladite aiguille intérieure (400) étant munie d'un corps cylindrique (410) ;
• lesdites canule (200) et aiguille de biopsie creuse (300) étant mutuellement mobiles de façon axiale,
**caractérisé en ce que** ledit ensemble formant aiguille de biopsie (100) comprend en outre une unité à vide couplée à ladite aiguille de biopsie creuse (300), dans lequel ladite unité à vide est une unité à vide à cylindre et piston constituée de ladite extrémité arrière cylindrique (302, 310) et dudit corps cylindrique (410), dans lequel ledit corps cylindrique (410) est mobile à l'intérieur de ladite extrémité arrière cylindrique (302, 310) de ladite aiguille de biopsie creuse (300) ;
dans lequel l'unité à vide est conçue pour créer une pression d'air abaissée dans ladite cavité intérieure (303) lorsque ladite extrémité avant (301) de ladite aiguille de biopsie creuse (300) est éloignée de façon axiale de ladite extrémité avant (401) et dans un sens vers l'avant par rapport à ladite extrémité avant (401) de ladite aiguille intérieure, l'unité à vide étant située de façon uniaxiale avec la canule (200) et l'aiguille de biopsie creuse (300).

2. Ensemble formant aiguille de biopsie (100) selon la revendication 1, dans lequel lesdites canule (200) et aiguille de biopsie creuse (300) et aiguille intérieure (400) sont mutuellement mobiles de façon axiale, ladite extrémité arrière (402) de ladite aiguille intérieure (400) s'étendant à partir de ladite cavité (303) de ladite aiguille de biopsie creuse (300) au niveau de l'extrémité arrière (302) de ladite aiguille de biopsie creuse (300), ladite cavité (303) de ladite aiguille de biopsie creuse (300) s'étendant dans ladite extrémité arrière cylindrique (310).

3. Ensemble formant aiguille de biopsie (100) selon la revendication 2, dans lequel ladite extrémité avant d'aiguille intérieure (401) est sensiblement située au niveau de l'extrémité extérieure de ladite aiguille de biopsie creuse (300) lorsque ledit corps cylindrique (410) est à sa position la plus proche près de ladite cavité (303).

4. Ensemble formant aiguille de biopsie (100) selon l'une quelconque des revendications 1 à 3, dans lequel ladite canule (200) comprend au moins une ouverture (204) dans sa surface extérieure pour enlever ledit échantillon de tissu dudit moyen de réception de tissu (304) de ladite aiguille de biopsie creuse (300) lorsque ledit moyen de réception de tissu (304) est amené devant l'ouverture (204) en déplaçant de façon axiale ladite aiguille de biopsie creuse (300) à l'intérieur de ladite canule (200).

5. Ensemble formant aiguille de biopsie (100) selon l'une quelconque des revendications 1 à 4, dans lequel ladite aiguille de biopsie creuse (300) est montée en rotation autour d'un axe commun (500) de la canule (200), de l'aiguille de biopsie creuse (300) et de l'aiguille intérieure (400).

6. Ensemble formant aiguille de biopsie (100) selon la revendication 5, dans lequel ladite extrémité arrière cylindrique (310) de ladite aiguille de biopsie creuse (300) est munie d'une roue de contact (315) pour mettre en prise une roue conjuguée afin de faire tourner ladite aiguille de biopsie creuse (300).

7. Ensemble formant aiguille de biopsie (100) selon l'une quelconque des revendications 5 ou 6, dans lequel ledit moyen de réception de tissu (304) est un moyen de réception de tissu formé en spirale (304).

8. Ensemble formant aiguille de biopsie (100) selon l'une quelconque des revendications 5 à 7, dans lequel ladite aiguille intérieure (400) est montée en rotation autour d'un axe commun (500) de la canule (200), de l'aiguille de biopsie creuse (300) et de l'aiguille intérieure (400).

9. Ensemble formant aiguille de biopsie (100) selon la revendication 8, dans lequel ledit corps cylindrique (410) ayant un filet de vis (412), le côté intérieur (311) de ladite extrémité arrière cylindrique (310) de ladite aiguille de biopsie creuse (300) comprenant un filet de vis (322) mettant en prise ledit filet de vis (412) dudit corps cylindrique (410), ledit corps cylindrique (410) comprenant un moyen (430) pour fournir un accouplement hermétique du corps cylindrique (410) et de l'extrémité arrière cylindrique (310).

10. Ensemble formant aiguille de biopsie (100) selon la revendication 8 ou 9, dans lequel ledit corps cylindrique (410) est muni d'une roue de contact (415) pour mettre en prise une roue conjuguée pour faire tourner ladite aiguille intérieure (400).

11. Ensemble formant aiguille de biopsie (100) selon l'une quelconque des revendications 1 à 10, dans lequel ladite canule (200) est munie d'une extrémité arrière cylindrique (210), la surface extérieure de ladite extrémité arrière cylindrique (310) de ladite aiguille de biopsie creuse (300) étant munie d'un filet de vis (312), le côté intérieur de ladite extrémité arrière cylindrique (210) de ladite canule (200) comprenant un filet de vis (212) mettant en prise ledit filet de vis (312) de ladite surface extérieure de ladite extrémité arrière cylindrique (310) de ladite aiguille de biopsie creuse (300).

12. Ensemble formant aiguille de biopsie (100) selon la revendication 11, dans lequel ladite canule (200) est montée en rotation autour d'un axe commun (500) de la canule (200), de l'aiguille de biopsie creuse (300) et de l'aiguille intérieure (400).

13. Ensemble formant aiguille de biopsie (100) selon la revendication 12, dans lequel ledit corps cylindrique de ladite surface extérieure de ladite extrémité arrière cylindrique (210) de ladite canule (200) est munie d'une roue de contact (215) pour mettre en prise une roue conjuguée pour faire tourner ladite canule (200).

14. Dispositif (600) pour prendre un échantillon de tissu au moyen d'un ensemble formant aiguille de biopsie (100) selon l'une quelconque des revendications 1 à 13, dans lequel ledit dispositif (600) comprend un moyen pour déplacer de façon axiale en avant et en arrière ladite aiguille de biopsie creuse (300), ledit dispositif (600) comprenant un moyen pour déplacer de façon axiale en avant et en arrière ladite canule (200), ledit dispositif comprenant un moyen pour empêcher l'aiguille intérieure (400) de se déplacer dans la direction axiale lorsque l'aiguille de biopsie creuse (300) est déplacée de façon axiale.

15. Dispositif (600) selon la revendication 14 pour prendre un échantillon de tissu au moyen d'un ensemble formant aiguille de biopsie (100) dans lequel ledit moyen pour déplacer de façon axiale en avant et en arrière ladite aiguille de biopsie creuse comprend un premier moyen d'entraînement couplé à une première et à une seconde roue conjuguée, ladite première roue conjuguée étant prévue pour mettre en prise et faire tourner ladite aiguille de biopsie creuse (300), ladite seconde roue conjuguée étant prévue pour mettre en prise et faire tourner simultanément ladite aiguille de biopsie creuse (300) et ladite aiguille intérieure (400), ledit moyen pour empêcher ladite aiguille intérieure (400) de se déplacer dans la direction axiale lorsque l'aiguille de biopsie creuse (300) est déplacée de façon axiale comprend un moyen pour empêcher ladite aiguille intérieure (400) de tourner lorsque ladite première roue conjuguée met en prise et fait tourner ladite aiguille de biopsie creuse (300).
